(19) Europäisches Patentamt / European Patent Office / Office européen des brevets

(11) **EP 2 794 730 B1**

(12) **FASCICULE DE BREVET EUROPEEN**

(45) Date de publication et mention
de la délivrance du brevet:
**18.04.2018 Bulletin 2018/16**

(21) Numéro de dépôt: **12816748.3**

(22) Date de dépôt: **20.12.2012**

(51) Int Cl.:
*C08J 3/20* *(2006.01)*     *C08L 67/04* *(2006.01)*
*C12N 9/14* *(2006.01)*     *C12N 11/04* *(2006.01)*
*C12N 11/08* *(2006.01)*

(86) Numéro de dépôt international:
**PCT/FR2012/053014**

(87) Numéro de publication internationale:
**WO 2013/093355 (27.06.2013 Gazette 2013/26)**

(54) **PROCÉDÉ DE PRÉPARATION D'ALLIAGE POLYMÈRE/ENTITÉS BIOLOGIQUES**

VERFAHREN ZUR HERSTELLUNG EINER MISCHUNG AUS POLYMER/BIOLOGISCHEN
WIRKSTOFFEN

METHOD FOR PREPARING A POLYMER/BIOLOGICAL ENTITIES BLEND

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.12.2011 FR 1162045**
**29.02.2012 FR 1251852**

(43) Date de publication de la demande:
**29.10.2014 Bulletin 2014/44**

(60) Demande divisionnaire:
**18160278.0**

(73) Titulaires:
• **Centre National de la Recherche Scientifique
(C.N.R.S.)**
**75794 Paris Cedex 16 (FR)**
• **Universite De Poitiers**
**86000 Poitiers (FR)**
• **Valagro Carbone Renouvelable
Poitou-Charentes**
**86000 Poitiers (FR)**

(72) Inventeurs:
• **FERREIRA, Thierry**
**F-86240 Iteuil (FR)**
• **BATAILLE, Frédéric**
**F-86022 Poitiers Cedex (FR)**
• **DEVER, Cédric**
**F-86022 Poitiers Cedex (FR)**
• **BARBIER, Jacques**
**F-86022 Poitiers Cedex (FR)**

(74) Mandataire: **Cabinet Becker et Associés
25, rue Louis le Grand
75002 Paris (FR)**

(56) Documents cités:
EP-A1- 2 325 255     WO-A1-2011/039489
JP-A- 4 168 149     JP-A- 2002 362 578

• TOKIWA YUTAKA ET AL: "Biodegradability of
plastics.", INTERNATIONAL JOURNAL OF
MOLECULAR SCIENCES SEP 2009 LNKD-
PUBMED:19865515, vol. 10, no. 9, septembre
2009 (2009-09), pages 3722-3742, XP002684789,
ISSN: 1422-0067
• EBATA H ET AL: "Lipase-catalyzed
transformation of poly(epsilon-caprolactone)
into cyclic dicaprolactone.", janvier 2000
(2000-01), BIOMACROMOLECULES WINTER
2000 LNKD- PUBMED:11710174, VOL. 1, NR. 4,
PAGE(S) 511 - 514, XP002684790, ISSN:
1525-7797 le document en entier
• MANDY ALISCH-MARK ET AL: "Increase of the
Hydrophilicity of Polyethylene Terephthalate
Fibres by Hydrolases from Thermomonospora
fusca and Fusarium solani f. sp. pisi",
BIOTECHNOLOGY LETTERS, SPRINGER
NETHERLANDS, DORDRECHT, vol. 28, no. 10, 23
May 2006 (2006-05-23), pages 681-685,
XP019391529, ISSN: 1573-6776, DOI:
10.1007/S10529-006-9041-7

EP 2 794 730 B1

- EBERL A ET AL: "Enzymatic hydrolysis of PTT polymers and oligomers", JOURNAL OF BIOTECHNOLOGY, ELSEVIER, AMSTERDAM, NL, vol. 135, no. 1, 20 May 2008 (2008-05-20), pages 45-51, XP022654399, ISSN: 0168-1656, DOI: 10.1016/J.JBIOTEC.2008.02.015 [retrieved on 2008-02-29]
- FRIEDRICH ET AL: "Ability of fungi to degrade synthetic polymer nylon-6", CHEMOSPH, PERGAMON PRESS, OXFORD, GB, vol. 67, no. 10, 16 March 2007 (2007-03-16), pages 2089-2095, XP005938435, ISSN: 0045-6535, DOI: 10.1016/J.CHEMOSPHERE.2006.09.038
- GATTIN RICHARD ET AL: "Biodegradation study of a starch and poly(lactic acid) co-extruded material in liquid, composting and inert mineral media", 2002, INTERNATIONAL BIODETERIORATION AND BIODEGRADATION, VOL. 50, NR. 1, PAGE(S) 25-31 ISSN: 0964-8305
- KAIHARA SACHIKO ET AL: "Enzymatic transformation of bacterial polyhydroxyalkanoates into repolymerizable oligomers directed towards chemical recycling.", 14 July 2005 (2005-07-14), MACROMOLECULAR BIOSCIENCE 14 JUL 2005, VOL. 5, NR. 7, PAGE(S) 644 - 652 ISSN: 1616-5187
- HEUMANN SONJA ET AL: "A Novel Aryl Acylamidase From Nocardia farcinica Hydrolyses Polyamide", BIOTECHNOLOGY AND BIOENGINEERING, vol. 102, no. 4, March 2009 (2009-03), pages 1003-1011, ISSN: 0006-3592(print)
- ISHIGAKI TOMONORI ET AL: "Enzymatic degradation of cellulose acetate plastic by novel degrading bacterium Bacillus sp. S2055", October 2000 (2000-10), JOURNAL OF BIOSCIENCE AND BIOENGINEERING, VOL. 90, NR. 4, PAGE(S) 400-405 ISSN: 1389-1723
- KUO P C ET AL: "Properties and biodegradability of chitosan/nylon 11 blending films", POLYMER DEGRADATION AND STABILITY, BARKING, GB, vol. 91, no. 12, 1 December 2006 (2006-12-01), pages 3097-3102, XP027949726, ISSN: 0141-3910 [retrieved on 2006-12-01]
- KLEEBERG ILONA ET AL: "Biodegradation of aliphatic-aromatic copolyesters by Thermomonospora fusca and other thermophilic compost isolates", May 1998 (1998-05), APPLIED AND ENVIRONMENTAL MICROBIOLOGY, VOL. 64, NR. 5, PAGE(S) 1731-1735 ISSN: 0099-2240
- "18" In: Negoro Seiji: "Biodegradation of Nylon and other synthetic polyamides", 15 January 2005 (2005-01-15) pages 395-401, DOI: 10.1002/3527600035.bpol9018,
- DATABASE BIOSIS [en ligne] BIOSCIENCES INFORMATION SERVICE, PHILADELPHIA, PA, US Mars 2007 NIMCHUA THIDARAT ET AL: 'Comparison of the hydrolysis of polyethylene terephthalate fibers by a hydrolase from Fusarium oxysporum LCH 1 and Fusarium solani f sp pisi' Database accession no. PREV200700309996 & NIMCHUA THIDARAT ET AL: "Comparison of the hydrolysis of polyethylene terephthalate fibers by a hydrolase from Fusarium oxysporum LCH 1 and Fusarium solani f sp pisi", BIOTECHNOLOGY JOURNAL, vol. 2, no. 3, March 2007 (2007-03), pages 361-364, ISSN: 1860-6768
- RONKVIST A M ET AL: "Cutinase-Catalyzed hydrolysis of poly(ethylene terephthalate)", 28 July 2009 (2009-07-28), MACROMOLECULES 20090728 AMERICAN CHEMICAL SOCIETY USA, VOL. 42, NR. 14, PAGE(S) 5128 - 5138
- NIMCHUA THIDARAT ET AL: "Comparison of the hydrolysis of polyethylene terephthalate fibers by a hydrolase from Fusarium oxysporum LCH 1 and Fusarium solani f sp pisi", March 2007 (2007-03), BIOTECHNOLOGY JOURNAL, VOL. 2, NR. 3, PAGE(S) 361-364 ISSN: 1860-6768
- YENG-FONG SHIH ET AL: "Enzymatic degradation kinetics of poly(butylene succinate) nanocomposites", JOURNAL OF POLYMER RESEARCH, KLUWER ACADEMIC PUBLISHERS-CONSULTANTS BUREAU, NL, vol. 16, no. 2, 12 June 2008 (2008-06-12), pages 109-115, XP019682721, ISSN: 1572-8935
- SILVA C M ET AL: "Cutinase - A new tool for biomodification of synthetic fibers", 1 June 2005 (2005-06-01), JOURNAL OF POLYMER SCIENCE, PART A: POLYMER CHEMISTRY 20050601 JOHN WILEY AND SONS INC. US, VOL. 43, NR. 11, PAGE(S) 2448 - 2450
- LEE CHAN WOO, KIMURA YOSHIHARU, CHUNG JIN-DO: "Mechanism of enzymatic degradation of poly(butylene succinate)", MACROMOLECULAR RESEARCH, vol. 16, no. 7, 1 October 2008 (2008-10-01), pages 651-658, ISSN: 2092-7673, DOI: 10.1007/BF03218575
- TANADCHANGSAENG NUTTAPOL ET AL: "Comonomer Compositional Distribution, Physical Properties, and Enzymatic Degradability of Bacterial Poly(3-hydroxybutyrate-co-3-hydroxy-4-methylvalerate) Copolyesters", June 2010 (2010-06), BIOMACROMOLECULES, VOL. 11, NR. 6, PAGE(S) 1615-1622 ISSN: 1525-7797(print)
- TSUJI HIDETO ET AL: "Enzymatic, alkaline, and autocatalytic degradation of poly(L-lactic acid): Effects of biaxial orientation", January 2006 (2006-01), BIOMACROMOLECULES, VOL. 7, NR. 1, PAGE(S) 380-387 ISSN: 1525-7797

**2**

- **ODA Y ET AL: "Degradation of Polylactide by Commercial Proteases", JOURNAL OF POLYMERS AND THE ENVIRONMENT, SPRINGER NEW YORK LLC, US, vol. 8, no. 1, 1 January 2000 (2000-01-01) , pages 29-32, XP002743808, ISSN: 1566-2543, DOI: 10.1023/A:1010120128048**
- **PULS JUERGEN ET AL: "Degradation of Cellulose Acetate-Based Materials: A Review", JOURNAL OF POLYMERS AND THE ENVIRONMENT, vol. 19, no. 1, March 2011 (2011-03), pages 152-165, ISSN: 1566-2543(print)**

Remarques:
Le dossier contient des informations techniques présentées postérieurement au dépôt de la demande et ne figurant pas dans le présent fascicule.

Remarques:
Le dossier contient des informations techniques présentées postérieurement au dépôt de la demande et ne figurant pas dans le présent fascicule.

**Description**

La présente divulgation concerne un procédé de préparation de matériaux polymères issus de l'industrie pétrochimique et/ou biosourcés comprenant dans leur composition des entités biologiques choisies parmi des enzymes et des microorganismes permettant de les dégrader.

Les matériaux polymères ont fait l'objet d'une utilisation intensive ces dernières années en particulier dans le domaine des plastiques. Cette exploitation intensive pour des usages courants s'est traduite par l'accumulation de plastiques dans notre environnement, source de nuisance visuelle, d'encombrement des décharges et de pollution des sols et des milieux maritimes. Aussi, du fait de leurs propriétés intrinsèques, notamment leur propriété de résistance à la dégradation, le traitement de déchets issus de ces matériaux constitue actuellement un réel problème environnemental et économique.

Il a été proposé plusieurs solutions, parmi lesquelles des matériaux plastiques biodégradables. Leur formulation vise en particulier à être adaptée à une dégradation par des microorganismes de l'environnement. Toutefois, cette dégradation s'effectue généralement de manière partielle. En outre, elle nécessite des conditions extrêmement favorables détaillées notamment dans la norme EN 13432. Ces conditions sont rencontrées dans des conditions artificielles, comme les composts industriels. Par exemple, ces matériaux sont généralement dégradés à des températures supérieures à 40°C. Ces conditions de température sont coûteuses à mettre en place, autant d'un point de vue énergétique que financier.

Les alternatives classiques pour le traitement de déchets, telles que l'incinération ou le dépôt en décharge, s'avèrent nuisibles même lorsqu'elles sont appliquées à des matériaux polymères biodégradables, leur dégradation ne s'effectuant pas de manière totale.

En outre, les matériaux biodégradables présentent des propriétés physiques altérées, notamment en termes de résistance à l'humidité, à la température et à l'élongation mécanique. Ces déficiences les rendent inadaptés à une utilisation dans les opérations classiques de plasturgie telles que l'injection ou l'extrusion et incompatibles avec les applications visées.

Ainsi, ces matériaux biodégradables, pourtant prometteurs, ne répondent pas aux exigences des industriels et aux exigences environnementales.

Il a été également proposé des matériaux constitués de polymères additionnés d'une charge végétale pour améliorer la dégradabilité desdits matériaux. Or, cette dégradation était due à la seule dégradation de ladite charge végétale, ce qui conduit nécessairement à une dégradation partielle. En outre, une telle solution s'est avérée insuffisante puisqu'elle ne permet pas la préservation des propriétés mécaniques du polymère. Un tel matériau est donc également limité quant à ses utilisations dans le domaine de la plasturgie.

Il existe donc un besoin pour des matériaux biodégradables, présentant des caractéristiques mécaniques équivalentes à celles des plastiques d'origine pétrochimique et adaptées pour leurs utilisations dans les opérations classiques de la plasturgie et, lesdits matériaux pouvant être dégradés totalement, à une vitesse de dégradation acceptable, et ceci dans des conditions de température, de pH et d'humidité compatibles avec celles généralement rencontrées dans l'environnement naturel.

A l'issue de recherches longues et approfondies, les inventeurs ont développé un procédé de préparation de matériaux polymères comprenant dans leur composition des entités biologiques permettant de les dégrader. Les matériaux polymères, ou alliages polymère/entités biologiques ainsi obtenus présentent des propriétés physicochimiques permettant la dégradation de manière totale en condition aqueuse tout en restant stable en condition solide. La présente divulgation concerne donc un procédé de préparation d'un alliage polymère/entités biologiques comprenant une étape de mélange d'un polymère et d'entités biologiques le dégradant lors d'un traitement thermique, ledit traitement thermique étant conduit à une température T supérieure à la température ambiante et lesdites entités biologiques étant résistantes à ladite température T, caractérisé en ce que lesdites entités biologiques sont choisies parmi les enzymes dégradant ledit polymère et les microorganismes dégradant ledit polymère. De manière surprenante et inattendue, les inventeurs ont montré qu'un tel procédé permet l'inclusion d'entités biologiques dans la structure même du polymère solide, tout en maintenant l'activité enzymatique desdites entités biologiques.

Sans vouloir être liés par la théorie, lorsque ladite entité biologique est une enzyme, les inventeurs avancent l'hypothèse selon laquelle l'élévation de la température s'accompagne d'une vaporisation des hydroxyles du polymère rendant l'enzyme active. Une telle activation de l'enzyme génère un début d'hydrolyse du polymère et donc une vitrification enrobant ladite enzyme. L'enzyme est alors protégée.

Les inventeurs ont également montré que la présence de l'entité biologique améliore sensiblement la dégradabilité dudit alliage, et ceci sans altérer les propriétés mécaniques du polymère. Aussi, les propriétés mécaniques dudit alliage sont très semblables, voire identiques aux propriétés mécaniques du polymère seul. Ces propriétés peuvent être déterminées à l'aide de la mesure de la résilience, de l'indice de fluidité à chaud, des paramètres de traction telles que la contrainte de traction maximale, l'allongement à la rupture ou encore le module de Young en traction. Aussi, l'alliage obtenu selon le procédé de l'invention est hautement approprié pour les opérations classiques de plasturgie.

De manière surprenante, les inventeurs ont montré que l'entité biologique maintient son activité enzymatique dans l'alliage de l'invention. En outre, ledit alliage reste stable lorsqu'il n'est pas en solution. L'entité biologique s'active donc uniquement lorsque l'alliage de l'invention est placé en solution. Aussi, la présence de ces entités biologiques permet le contrôle des conditions et de la vitesse de dégradation de l'alliage de l'invention.

Les alliages de l'invention ont donc l'avantage d'être stables quand ils ne sont pas placés en solution, ce qui d'une part facilite le stockage et le transport du matériau et d'autre part indique l'existence d'un effet "release" (ou effet retard) de l'activation de l'entité biologique. Un tel effet release est très avantageux puisqu'il permet de contrôler l'activation de l'activité enzymatique et de la dégradation de l'alliage de l'invention.

Par **"alliage polymère/entités biologiques"** ou **"alliage de l'invention",** on entend un polymère comprenant dans sa composition des entités biologiques permettant de les dégrader. Cette expression englobe donc les alliages polymère/enzymes et les alliages polymère/microorganismes.

Par **"entité biologique"** on entend une enzyme ou un microorganisme. Dans le contexte de la présente invention, ladite entité biologique a pour caractéristique la possibilité de dégrader un polymère d'intérêt.

Par **"alliage polymère/enzymes",** on entend un polymère comprenant dans sa composition des enzymes permettant de les dégrader. Préférentiellement, ledit polymère est un polymère biodégradable. L'utilisation d'un polymère biodégradable comme matériel de départ pour la mise en oeuvre du procédé permet d'obtenir un alliage polymère/enzymes présentant des propriétés de dégradation plus avantageuses.

Par **"alliage polymère/microorganismes"**, on entend un polymère comprenant dans sa composition des microorganismes permettant de le dégrader. Typiquement, ces microorganismes produisent des enzymes dégradant ledit polymère.

Par "**traitement thermique**", on entend toutes opérations de transformation de polymère lors d'une élévation de la température dudit polymère, préférentiellement à une température supérieure à la température ambiante, et plus préférentiellement à une température supérieure à 50°C. Préférentiellement, ledit traitement thermique permet l'inclusion de l'entité biologique. Plus préférentiellement, ledit traitement thermique consiste en une opération choisie parmi l'extrusion, l'injection, le thermoformage, le rotomoulage, la compression, le calandrage, la chaudronnerie, l'enduction, la stratification, l'expansion, la pultrusion, l'extrusion-soufflage, l'extrusion-gonflage, et la compression-granulation. Ces opérations peuvent être mises en oeuvre sur des polymères à l'état liquide et/ou solide. Dans un mode de réalisation préféré, le polymère est à l'état solide. Dans un autre mode de réalisation, ledit polymère est sous forme de sirop.

Par "**température ambiante**", on entend généralement une température comprise entre 20°c et 30°, plus préférentiellement une température d'environ 25°C.

Par "**extrusion**", on entend la préparation d'un polymère sous une forme désirée, à partir d'un matériau sous forme de granulés ou de poudre, et ceci à l'aide d'une extrudeuse. Cette expression englobe l'extrusion profilée, l'extrusion soufflage, l'extrusion gonflage, l'extrusion calandrage.

Cette étape d'extrusion s'effectue à la température de fusion dudit polymère. Le polymère est alors dans un état fondu partiellement ou en totalité. Cette température est donc dépendante de la nature dudit polymère et de la nature de l'entité biologique d'intérêt. Cette température peut facilement être déterminée par l'homme du métier, à la lumière de ses connaissances générales. Sous l'action de la température et de la pression, ledit polymère biodégradable à l'état fondu ou partiellement fondu va se mélanger aux autres matières premières, et notamment aux entités biologiques le dégradant. Préférentiellement, ladite température T est comprise entre la température de transition vitreuse et la température de fusion dudit polymère. Plus préférentiellement, ladite température T est la température de fusion dudit polymère. Typiquement, ladite température T dépend de la nature dudit polymère et la nature de l'entité biologique d'intérêt et est supérieure à 50°C, préférentiellement supérieure à 60°C, préférentiellement supérieure à 70°, préférentiellement supérieur à 80°, préférentiellement supérieure à 90°, préférentiellement supérieure à 100°, préférentiellement supérieure à 120°, préférentiellement supérieure à 140°C, préférentiellement supérieure 150°C. Typiquement, cette température T ne dépasse pas 300°C.

La principale fonction de l'extrudeuse est de permettre par l'action de la température et de la pression, le passage du polymère à travers une filière qui se trouve à son extrémité. Typiquement, une extrudeuse est composée d'un ou plusieurs fourreaux chauffants présentant différent grades de température, d'une ou de deux vis d'Archimède qui vont permettre le transport de la matière le long du fourreau, d'une trémie qui permet l'alimentation en différents points de la matière à extruder qui se présente sous la forme de granulés ou de farine, d'une filière plus ou moins complexe qui se trouve au bout du fourreau et qui permet de donner la forme et la taille désirée à la matière plastique qui sort en continu. Préférentiellement, l'étape d'extrusion se fait à l'aide d'une extrudeuse de type bi-vis BC21, commercialisée sous la dénomination CLEXTRAL.

La mise en oeuvre de cette étape d'extrusion entre dans les compétences normales de l'homme du métier. Elle se fait généralement selon les étapes suivantes :

- introduction d'un mélange de polymère et d'entités biologiques le dégradant ;
- passage dudit mélange dans l'extrudeuse ;

- sortie d'un jonc à travers une filière ;
- refroidissement dudit jonc éventuellement suivi d'un séchage ;
- découpe sous forme de granules réguliers en fonction de la forme souhaitée ; et
- séchage, préférentiellement dans une turbétuve, à une température comprise entre environ 40°C et environ 60°C, encore plus préférentiellement à une température d'environ 50°C.

Typiquement, le ratio entités biologiques/polymère en poids est compris entre environ 0,1% et environ 10%, préférentiellement entre environ 0,5% et environ 8%, préférentiellement entre environ 1% et environ 6%, préférentiellement entre environ 1% et environ 5%, préférentiellement entre environ 1% et environ 4%, préférentiellement entre environ 1% et environ 3%, préférentiellement entre environ 1,5% et environ 3%, et encore plus préférentiellement ce ratio est d'environ 2%. Ce ratio est adapté par l'homme du métier en fonction de la nature du polymère biodégradable, la nature des entités biologiques utilisées, et les résultats recherchés, notamment en termes de dégradabilité de l'alliage obtenu par le procédé.

Préférentiellement, le procédé comprend en outre l'ajout d'une substance susceptible d'optimiser les capacités de dégradation desdites entités biologiques. Typiquement, lorsque lesdites entités biologiques sont des enzymes, ces substances peuvent être des cofacteurs desdites enzymes, comme des cations divalents.

Dans le contexte de la présente divulgation l'expression "**polymère**" englobe les polymères issus de l'industrie pétrochimique, les polymères biosourcés, et les polymères biosurfaits.

Dans un mode de réalisation particulier, les polymères pertinents dans le contexte de la présente divulgation sont issus de l'industrie pétrochimique. L'intérêt de ces polymères est la maîtrise du processus de polymérisation et des constituants de base qui permettent de garantir une transformation aisée. Typiquement, il s'agit de polymères renfermant des unités monomères comprenant des liaisons hydrolysables, comme les esters ou les amides.

Dans un autre mode de l'invention, les polymères pertinents dans le contexte de la présente invention sont les polymères biosourcés.

Par "**polymère biosourcé**" on entend un polymère issu de ressources renouvelables. Ces polymères biosourcés sont parfois utilisés en combinaison avec des additifs tels que des plastifiants. Ledit polymère est choisi parmi le polycaprolactone (ou CAPA), l'acide polylactique, le polyéthylène téréphtalate, le poly(triméthylène téréphthalate) un polyhydroxyalkanoate en $C_1$-$C_6$, l'acétate de cellulose, le poly(butylène adipate-co-téréphtalate), le poly(butylene succinate), le polyamide PA11 et leurs mélanges.

Plus préférentiellement, ledit polymère est l'acide polylactique ou PLA. Le PLA a des propriétés mécaniques semblables à certains thermoplastiques pétrochimiques tels que le polypropylène.

Encore plus préférentiellement, ledit polymère est le polycaprolactone ou CAPA.

Dans le cadre de la présente invention, il convient de choisir une entité biologique, préférentiellement une enzyme qui résiste à la température de mise en oeuvre de l'étape d'extrusion selon le procédé de l'invention. L'homme du métier dispose des connaissances générales lui permettant de déterminer ces températures et d'identifier des entités biologiques qui résistent à ces températures.

Par "**polymère biodégradable**", on entend un matériau susceptible d'être dégradé par des entités biologiques. Le résultat de la dégradation de tels matériaux est la formation d'eau, de dioxyde de carbone et de méthane, et éventuellement de sous-produits. Les sous-produits obtenus lors de la dégradation desdits polymères sont non toxiques.

La norme NE 13432:2000 énonce les exigences relatives aux emballages valorisables par compostage et biodégradation. Elle fixe les caractéristiques qu'un matériau doit présenter pour être défini comme compostable. Elle se base sur les critères suivants :

➢ La biodégradabilité : elle est mesurée par la conversion métabolique du matériau en dioxyde de carbone. Cette propriété est mesurée en utilisant la méthode standardisée EN 14046. Le taux de décomposition à atteindre est de 90 % en moins de 6 mois.

➢ La désintégration : Il s'agit de la fragmentation du matériau et de son absence d'identification visuelle dans le compost final. Elle est mesurée par la méthode de compostage EN 14045. Le matériau doit être désagrégé en présence de déchets organiques en moins de trois mois. Après cette durée, le compost est tamisé avec un tamis de 2 mm. Les résidus du matériau possédant une taille supérieure à 2 mm sont considérés comme non-désintégrés. Cette fraction doit représenter moins de 10 % de la masse initiale.

➢ Un faible taux de métaux lourds et l'absence d'effets négatifs sur la qualité du compost: un test de croissance de plantes est réalisé, selon la méthode OECD test 208, avec un échantillon de compost. D'autres paramètres physico-chimiques du compost ne doivent pas être modifiés (par rapport à un compost ne contenant pas de polymères) : salinité, % d'azote, de phosphore, de magnésium et de potassium.

Dans un premier mode de réalisation préféré, lesdites entités biologiques sont des enzymes, préférentiellement des enzymes résistantes à la température d'extrusion.

Par **"enzymes résistantes à la température d'extrusion"**, on entend des enzymes dont la structure protéique et/ou l'activité enzymatique ne sont pas affectées par la température à laquelle l'étape d'extrusion selon le procédé de l'invention est effectuée. Ces enzymes sont donc hautement appropriées pour une utilisation à des températures supérieures à la température ambiante.

Préférentiellement, lesdites enzymes sont choisies parmi les enzymes thermorésistantes et les enzymes thermostabilisées.

Par "**enzymes thermorésistantes**", on entend des enzymes dont la nature intrinsèque procure une résistance aux températures élevées, en particulier à la température à laquelle l'étape d'extrusion selon le procédé de l'invention est effectuée.

Préférentiellement, lesdites enzymes thermorésistantes sont choisies parmi la lipase PS de *Pseudomonas cepacia,* la lipase B de *Candida antartica,* la protéinase K, une polyhydroxyalkanoate en $C_1$-$C_6$ dépolymérase et leurs mélanges.

Par enzymes **"thermostabilisées"** ou **"thermoprotégées"**, on entend des enzymes qui ne sont pas naturellement thermorésistantes mais qui se présentent sous une forme particulière leur conférant une résistance à la température à laquelle l'étape d'extrusion selon le procédé de l'invention s'effectue. Préférentiellement, ces enzymes thermostabilisées sont obtenues par un procédé chimique ou physique.

Préférentiellement, lesdites enzymes thermostabilisées sont choisies parmi les enzymes encapsulées dans des nanocapsules constituées de la même matière que ledit polymère, les enzymes encapsulées dans des molécules cages et les enzymes agrégées entre elles.

Ces enzymes thermostabilisées peuvent être obtenues par encapsulation des enzymes non thermorésistantes dans des nanocapsules, préférentiellement des nanocapsules constituées de la même matière que ledit polymère. Les techniques permettant l'encapsulation sont bien connues de l'homme du métier. Typiquement, cette encapsulation s'effectue par la mise en oeuvre de nanoémulsions. Cette encapsulation des enzymes permet le contrôle de l'activation des enzymes. Ce mode de réalisation est particulièrement avantageux pour une utilisation des alliages de l'invention dans l'emballage des produits alimentaires.

Ces enzymes thermostabilisées peuvent également être obtenues par encapsulation des enzymes dans des molécules cage. Une telle encapsulation permet de protéger lesdites enzymes d'une quelconque dégradation liée à la température.

Par "**molécule cage**" on entend une molécule susceptible de s'insérer dans la structure desdites enzymes pour les stabiliser et les rendre résistantes à des températures supérieures à la température ambiante.

Ces enzymes thermostabilisées peuvent également être obtenues par agrégation entre elles des enzymes non thermorésistantes. L'homme du métier dispose des connaissances techniques suffisantes pour mettre en oeuvre une telle agrégation.

Dans un mode de réalisation particulier, lesdites enzymes sont sous forme d'apoenzymes, et sont activées en présence de cofacteurs.

Dans un second mode de réalisation, lesdites entités biologiques sont des microorganismes.

Typiquement, ces microorganismes sont des microorganismes susceptibles ou non de sporuler et produisant des enzymes dégradant des polymères d'intérêt. Préférentiellement, ces microorganismes sont des bactéries, des champignons ou des levures.

Dans ce mode de réalisation, l'alliage de l'invention est un alliage polymère/microorganismes, préférentiellement un alliage acide polylactique/microorganismes.

Préférentiellement, lesdits microorganismes sont choisis parmi les bactéries du genre *Ochrobactrum,* les bactéries appartenant au phylum des Firmicutes (*Firmus cutis*), les bactéries de la classe des bacilles, notamment les souches de bactéries *Bacillus cereus spp.* et plus particulièrement *Bacillus cereus G9241,* et les souches de bactéries *Bacillus clausii spp.*

Plus préférentiellement, ledit microorganisme est une souche de bactérie du genre *Ochrobactrum,* dénommée *Ochrobactrum* sp. 37S, et déposée selon la Traité de Budapest le 23 juillet 2009 au nom du Centre National de la Recherche Scientifique à la Collection Nationale de Cultures de Microorganismes sous le numéro CNCM I-4212, ou un variant de ladite souche, ledit variant étant capable de dégrader l'acide polylactique.

Préférentiellement, ledit polymère est l'acide polylactique et lesdites entités biologiques sont des bactéries *Ochrobactrum* sp. 37S, lesdites bactéries dégradant l'acide polylactique.

Par "**variant**", on entend :

➢ un variant naturel d'une souche, c'est à dire un variant obtenu spontanément à partir d'une souche après incubation dans un milieu de sélection. Un variant naturel est donc obtenu sans aucune manipulation génétique de l'opérateur mais seulement par mutation naturelle de la souche et sélection de cette souche mutée dans un milieu approprié, ou
➢ un variant d'une souche comprenant au moins une mutation dans son génome, ladite mutation étant induite par génie-génétique, par exemple par mutagénèse dirigée ou mutagénèse aléatoire. Par exemple, la mutagénèse aléatoire peut être effectuée à l'aide de mutagènes tels que les radiations (rayons UV, radiations ionisantes, chaleur) ou des composés chimiques (acide nitreux, éthyl-méthanesulfonate, N-méthyl-N'-nitro-N-nitrosoguanine, N-éthyl-N-nitrosourea, acridine orange, proflavine, etc.).

Par "**mutation**", on entend l'addition, la délétion ou la substitution d'au moins un nucléotide dans le génome de la souche selon l'invention.

Aussi, l'invention concerne un procédé de préparation d'un alliage polymère/entités biologiques comprenant les étapes suivantes:

i) sélection d'un polymère parmi le polycaprolactone, l'acide polylactique, le polyéthylène téréphtalate, le poly(triméthylène téréphthalate), un polyhydroxyalkanoate en C1-C6, l'acétate de cellulose, le poly(butylène adipate-co-téréphtalate), le poly(butylène succinate), le polyamide PA 11, et leurs mélanges

ii) sélection d'entités biologiques susceptibles de dégrader ledit polymère et résistantes à une température T supérieure à la température ambiante ; et

iii) mélange dudit polymère et desdites entités biologiques lors d'un traitement thermique conduit à la température T,

caractérisé en ce que lesdites entités biologiques sont choisies parmi les enzymes dégradant ledit polymère et les microorganismes dégradant ledit polymère.

Toutes les caractéristiques décrites précédemment s'appliquent à ce procédé.

Le procédé a pour avantage d'être compatible avec les équipements classiquement utilisés dans l'industrie de la plasturgie, ce qui permet sa mise en oeuvre rapide et directe par les professionnels, sans modification significative des outils de production classiquement utilisés, notamment ceux ordinairement réservés à l'élaboration de plastiques d'origine pétrochimique.

En outre, le procédé de l'invention ne nécessite pas l'utilisation de produits potentiellement dangereux pour la santé humaine ou l'environnement. Il ne génère pas non plus de sous-produits pouvant représenter un tel danger.

Le procédé permet d'obtenir un alliage polymère/entités biologiques "à façon" en incluant des entités biologiques, préférentiellement des enzymes, à un polymère, préférentiellement un polymère biodégradable, ce qui conditionne la dégradation des alliages ainsi obtenus. La présente divulgation permet donc d'obtenir un alliage polymère/entités biologiques, préférentiellement un alliage polymère/enzymes unique dont il est possible de contrôler la vitesse de dégradation.

Une liste non limitative de ces alliages polymère/enzymes est constituée des couples polycaprolactone/lipase PS de *Pseudomonas cepacia* ; polycaprolactone/lipase B de *Candida antartica* ; acide polylactique/Protéinase K ; polyhydroxyalkanoate en $C_1$-$C_6$/polyhydroxyalkanoate en $C_1$-$C_6$ dépolymérase.

Préférentiellement, l'invention concerne un procédé de préparation d'un alliage polymère/enzymes comprenant l'extrusion du polycaprolactone avec une lipase, préférentiellement l'amanolipase PS de *Pseudomonas cepacia,* à la température de fusion du polycaprolactone dans un ratio enzymes/polymère de 2% en poids. En effet, les inventeurs ont mis en évidence le fait que l'alliage ainsi obtenu se dégrade totalement au bout de 4 mois en milieu aqueux, tandis que le polycaprolactone non supplémenté en lipase ne se dégrade pas sur cet intervalle de temps.

Les inventeurs ont en outre montré et exemplifié le fait qu'un tel alliage se dégrade à des températures d'extrusion de 80°C, 100°C, 120° C et 140°C. Il est également divulgué un alliage polymère/enzymes, caractérisé en ce que lesdites enzymes dégradent ledit polymère et qu'elles sont thermostabilisées.

Ces alliages sont hautement avantageux du fait d'une part de leur dégradabilité améliorée lorsqu'ils sont placés en solution aqueuse et d'autre part de leurs propriétés mécaniques adaptées à leurs utilisations dans l'industrie. L'inclusion de l'enzyme au sein de l'alliage n'altère pas les propriétés du polymère seul. Aussi, les propriétés mécaniques dudit alliage sont très semblables, voire identiques aux propriétés mécaniques du polymère seul.

Préférentiellement, lesdites enzymes thermostabilisées sont choisies parmi les enzymes encapsulées dans des nanocapsules constituées de la même matière que ledit polymère, les enzymes encapsulées dans des molécules cages et les enzymes agrégées entre elles.

Toutes les caractéristiques techniques précédemment énoncées concernant ledit polymère et lesdites enzymes thermostabilisées sont applicables à cet alliage.

Alternativement, il est divulgué un alliage polymère/entités biologiques caractérisé en ce que ledit polymère est l'acide polylactique et lesdites entités biologiques sont des bactéries *Ochrobactrum* sp. 37S, lesdites bactéries dégradant l'acide polylactique.

Dans un autre aspect, il est divulgué l'utilisation de l'alliage polymère/entités biologiques dans les secteurs de l'agriculture, l'horticulture, l'emballage, la restauration, l'environnement, le transport, le textile, l'électronique et la pharmacie. Dans la suite, les expressions "alliage "et "alliage polymère/entités biologiques obtenus selon le procédé de l'invention" sont utilisées indifféremment.

Préférentiellement, il est divulgué l'utilisation de l'alliage dans le secteur de l'emballage, plus préférentiellement pour l'emballage de produits alimentaires, notamment de fruits et légumes et en boulangerie. Aussi, il est divulgué l'utilisation de l'alliage en tant qu'emballage alimentaire à longue durée de conservation. En effet, l'alliage est hautement adapté pour une mise en contact avec des produits alimentaires puisqu'il ne présente pas d'effets néfastes, qu'il est aisément dégradable et qu'il constitue une barrière au passage du dioxyde de carbone, de l'oxygène et de l'eau, évitant ainsi l'altération des aliments. L'utilisation de l'alliage dans le secteur de l'agriculture, notamment pour l'obtention de granulés comprenant des produits phytosanitaires, de films de paillage, de films de tunnel et de matériels de palissage est également divulguée. En outre l'utilisation dudit alliage pour l'obtention de bouteilles est divulguée. Enfin, l'utilisation de l'alliage dans le domaine médical, notamment pour la production de sutures résorbables et comme vecteur de molécule thérapeutiquement active est divulguée. Préférentiellement, ces alliages sont utilisés sous la forme de nanoparticules renfermant ladite molécule thérapeutiquement active. L'homme du métier dispose des connaissances techniques générales lui permettant de préparer des nanoparticules à partir de l'alliage. L'intérêt de l'utilisation des alliages en thérapie est évident à plusieurs niveaux. D'une part, la dégradation de la nanoparticule permet de délivrer le médicament de façon progressive dans les cellules cibles. En outre, elle permet de limiter les effets secondaires liés à l'accumulation de la molécule thérapeutique dans certains tissus, tels que les organes de détoxification comme le foie et la rate. Enfin, la biodégradabilité de telles nanoparticules va limiter les impacts potentiels sur l'environnement, qui sont associés à la diffusion des nanoparticules dans le milieu extérieur via les secrétions naturelles du patient.

Le procédé de préparation d'alliages selon l'invention permet d'obtenir des alliages présentant une dégradabilité adaptée en fonction de l'utilisation souhaitée. Aussi, dans un contexte thérapeutique, l'homme du métier sera capable d'adapter le procédé pour obtenir un alliage n'entrainant pas d'accumulation de nanoparticules dans certains organes du patient traité, afin de pouvoir éviter les effets indésirables qui peuvent être associés à la présence desdites nanoparticules.

L'homme du métier devra en outre adapter la nature de la molécule thérapeutiquement active encapsulée par la nanoparticule en fonction du type de pathologies à traiter et de la nature de la nanoparticule *per se.* En effet, en fonction des caractéristiques physicochimiques de ladite nanoparticule (notamment son poids, sa taille, sa lipophilie, son hydrophilie, et son état d'ionisation), elle est susceptible ou non de traverser la barrière transmembranaire. Ainsi, la nature de la nanoparticule influe sur la distribution de la molécule thérapeutiquement active chez l'individu chez lequel la nanoparticule constitué de l'alliage est administrée.

**Exemples:**

**Exemple 1: Préparation d'alliages polymère/enzymes selon l'invention**

**Matériels et Méthodes**

1. Inclusion d'enzymes dans le matériau de départ lors de l'étape d'extrusion

L'incorporation d'enzymes dans le polymère biodégradable de départ est réalisée lors de l'étape dite d'extrusion.

L'étape d'extrusion est réalisée grâce à une extrudeuse de type bi-vis BC21 de marque CLEXTRAL (puissance moteur 9 kW, vitesse vis max 600 rpm, ampérage max 18.9 A). Les vis ont un diamètre d de 25 mm et l'écart entre les deux vis est de 21 mm. La longueur du fourreau est de 600mm soit un rapport L/d de 24.

L'extrusion se fait en 5 étapes :

- introduction d'un mélange polymère biodégradable/enzymes,
- passage dudit mélange dans l'extrudeuse,
- sortie d'un jonc à travers une filière circulaire de 3 mm de diamètre,
- refroidissement du jonc dans un bac d'eau froide de trois mètres de long puis "séchage" par de l'air froid pulsé,
- découpe sous forme de granules réguliers par un système avec couteau tournant.

Les formulations peuvent varier en fonction du rapport polymère biodégradable/enzymes. Dans les expériences présentées dans cette demande, les résultats correspondent à un rapport de 2 % (m/m) d'enzyme dans le matériau.

Les granulés obtenus par extrusion sont ensuite mis à sécher en turbétuve à 50°C pendant 15 heures (turbo-mélangeur équipé d'une double enveloppe à circulation d'huile) afin d'éliminer l'eau résiduelle présente, due au passage dans le bac à eau. Le suivi du taux d'humidité lors du séchage est réalisé grâce à un analyseur d'humidité équipé de résistances infrarouge.

2) Préparation des éprouvettes - Injection

Afin d'évaluer les propriétés mécaniques de l'alliage polymère/enzymes obtenu, ainsi que ses capacités de dégradation, des pièces moulées de format standard, appelées « éprouvettes », sont obtenues par injection.

L'injection est un procédé discontinu permettant la mise en forme de granulés en produits finis tridimensionnels. Le principe consiste à chauffer la matière plastique dans le fourreau d'alimentation pour l'amener à l'état fondu, vers la zone de dosage et de compression pour l'injecter dans le moule par l'intermédiaire d'un piston. La pièce moulée est refroidie dans le moule, puis éjectée.

La presse à injecter qui a été utilisée est de marque ARBURG, de modèle Allrounder 1000-420C-250. Les éprouvettes injectées correspondent au type 1 de la norme ISO 3167.

## Résultat

### 1. Sélection des enzymes en milieu liquide

Pour la mise en oeuvre du procédé, il convient d'identifier des enzymes capables de dégrader le polymère biodégradable, afin d'identifier le bon « couple » enzymes/matériau qui sera utilisé lors de l'étape d'extrusion.

Nous avons développé deux tests simples afin d'identifier les enzymes d'intérêt. Ces tests sont réalisés sur des échantillons de matériau pur sous forme d'éprouvette ou de granulés, respectivement.

Les tests présentés dans ce qui suit ont été réalisés sur des éprouvettes ou des granulés de polycaprolactone ou CAPA (Perstorp ; Ref. 6506 ; 50.000 g/mol). Les inventeurs ont évalué deux enzymes commerciales, connues pour leur capacité à dégrader le CAPA : la Lipase PS de *Pseudomonas cepacia* et la Lipase AK de *Pseudomonas fluorescens* (Amano, Japon).

*a) Tests sur éprouvettes*

Les éprouvettes de polymères vierges mises en biodégradation sont des éprouvettes issues de l'injection des granulés de CAPA. Tous les tests de biodégradation se déroulent dans une étuve et thermorégulée à 30 °C.

Les tests présentés dans cette étude ont été réalisés dans une solution nutritive, dont la composition est définie selon la norme ISO 14852 : 1999. Alternativement, la dégradation peut également être évaluée en eau de marque *Fontaine Cristalline,* c'est-à-dire une eau de source de composition constante permettant d'avoir les mêmes conditions de milieu.

Pour ces tests, les éprouvettes sont maintenues entièrement immergées dans la solution, additionnée ou non d'enzymes à la concentration finale de 200 mg/L.

Le suivi de la dégradation des matériaux est réalisé par calcul de la perte de masse de l'échantillon après un temps défini de présence dans le milieu.

Cette méthode utilise une série de 13 éprouvettes identiques, cinq d'entre elles étant étuvées à 103 °C pendant 48 heures afin de déterminer la perte en eau de l'échantillon. Cette étape permet d'effectuer une correction pour le calcul de perte de masse des échantillons placés en biodégradation. Les 8 autres éprouvettes sont immergées dans le milieu de dégradation et placées dans une étuve thermorégulée à 30°C. Elles seront ensuite prélevées au bout d'un temps défini afin de suivre le comportement du matériau au cours du temps. Après chaque prélèvement, les éprouvettes sont séchées à l'étuve à 103 °C pendant 48 heures et pesées.

La perte de masse est calculée selon la formule suivante :

$$\Delta m = \frac{(m_i - m_f)}{m_i} * 100 - \Delta m \text{ entre température ambiante et } 103°C$$

Avec $m_i$ : masse initiale de l'éprouvette
$m_f$ : masse finale de l'éprouvette

Les résultats montrent que la présence de la lipase de *Pseudomonas fluorescens* ne permet pas d'améliorer la biodégradabilité de la polycaprolactone puisque le taux de dégradation est sensiblement le même que lorsque les enzymes ne sont pas présentes.

Par contre, en ce qui concerne la lipase de *Pseudomonas cepacia,* on constate une très nette différence dès le premier prélèvement réalisé après 7 jours de contact avec le milieu. En effet, un taux de dégradation de 5 % est obtenu alors que dans le même temps l'échantillon dégradé en l'absence d'enzyme ne présente qu'une perte de masse de 0,2 %. Néanmoins, un palier est rapidement observé et le taux de biodégradation n'évolue plus. Il a donc été décidé de changer la solution nutritive additionnée d'enzymes (toujours à 200 mg/L de solution nutritive). Ce changement a été effectué 44 jours après le début de la dégradation. Ceci a permis d'observer une reprise de la dégradation jusqu'à environ 9 % en 70 jours.

*b) Tests sur granules*

L'avantage de cette méthode est qu'elle permet de travailler sur de petits volumes réactionnels, rendant les expériences sensiblement moins coûteuses. De plus, plusieurs enzymes et plusieurs concentrations peuvent être testées simultanément. Enfin, la possibilité de travailler avec des concentrations d'enzymes élevées permet d'obtenir des taux de dégradation importants sur des temps courts.

Dans cette expérience, les granules de CAPA (volume approximatif : 50 mm$^3$ ; masse approximative : 40 mg) ont été incubés dans 1,9 mL d'un tampon (Tampon Phosphate 25 mM, pH7, Azide de Sodium 2 g/L) additionné, ou non, de Lipase PS de *Pseudomonas cepacia* à la concentration finale de 20 mg/mL. L'incubation est réalisée à 28°C. A intervalle de temps réguliers, les granulés sont prélevés, rincés abondamment à l'eau avant d'être incubés pendant 24 h à 28°C pour séchage. La masse est alors déterminée et comparée à la masse de départ, c'est-à-dire avant incubation dans le mélange réactionnel.

Les résultats montrent un taux de dégradation du CAPA de plus de 50 % avec la Lipase PS de *Pseudomonas cepacia,* dans les conditions précisées, confirmant les résultats obtenus avec les éprouvettes.

Ce protocole peut être utilisé pour la détermination de l'enzyme la plus pertinente en vue de son inclusion ultérieure dans un polymère donné.

De plus, il peut également permettre d'identifier certains « additifs » ayant la propriété d'optimiser l'activité de dégradation. Ces additifs pourraient alors être additionnés avec l'enzyme dans le matériau lors de l'étape d'extrusion, pour en accélérer la dégradation.

## 2. Evaluation de la résistance thermique des enzymes afin de déterminer la température optimale d'extrusion

Lors de l'étape d'extrusion, le mélange polymère/enzymes est soumis à une température élevée correspondant à la température de fusion du polymère. Dans le cas du CAPA, nous avons fixé cette température d'extrusion à 80°C. Selon la fiche technique du fournisseur, la lipase PS de *Pseudomonas cepacia* est stable sous forme de poudre pendant plusieurs heures à 100°C.

Néanmoins, ces données se réfèrent à l'activité lipase de l'enzyme et n'évaluent pas la résistance thermique de son activité CAPA dépolymérase.

Nous avons donc réalisé l'expérience sur granulés décrite ci-dessus, mais en traitant la solution enzymatique (20 mg/mL d'enzyme) pendant 5 min à 80 °C. Le temps et la température sélectionnés correspondent à des conditions thermiques proches celles rencontrées lors de l'étape d'extrusion.

Les résultats montrent que ce traitement a un faible impact sur l'activité CAPA dépolymérase de l'enzyme. En effet, les inventeurs ont mis en évidence uniquement 10 % de perte d'activité à 7 jours. Il s'ensuit que la lipase PS de *Pseudomonas cepacia* est un excellent candidat pour la réalisation de l'alliage CAPA/enzyme.

## 3. Incorporation de la lipase PS de *Pseudomonas cepacia* lors du processus de mise en oeuvre de la polycaprolactone

Les inventeurs ont donc réalisé une extrusion de la polycaprolactone en incorporant ladite enzyme. Pour cela un mélange de polymère sous forme de poudre et d'enzyme sous forme de poudre est placé dans un doseur.

Dans les expériences qui sont présentées ici, les débits de ces deux doseurs ont été réglés de manière à obtenir un pourcentage d'enzyme dans le matériau de **2 %** (m/m). Une formulation sans enzyme a également été préparée (contrôle) (Tableau 1).

Tableau 1 : Formulations réalisées dans cette étude

| Formulation (% massique) | Masse d'enzyme (g) dans doseur | Masse CAPA 6506 P (g) dans doseur |
|---|---|---|
| 98%m CAPA 6506 + 2%m lipase | 30 | 1500 |
| 100%m CAPA 6506 | 0 | 1500 |

L'extrusion a été réalisée à 80°C, de manière à avoir une température supérieure au point de fusion du CAPA (60°C), mais pas trop élevée, afin d'éviter tout risque de dénaturation de l'enzyme.

Les paramètres précis de l'extrusion, qui a été réalisée dans les mêmes conditions pour les deux formulations, sont présentés dans le Tableau 2.

Tableau 2 : Paramètres des extrusions réalisées dans cette étude

| Formulation | Températures d'extrusion (Zone 1 à filière) | Vitesse BC 21, T/min | Pression, bars | Couple (%) |
|---|---|---|---|---|
| 98%m CAPA 6506 + 2%m lipase | 18/20/39/65/67/72/68/6 5/71 | 256 | 71-74 | 53-54 |
| 100%m CAPA 6506 | 20/20/40/65/65/69/65/6 5/65 | 256 | 73-75 | 55-60 |

Le terme "couple" cité dans le tableau 2 correspond à l'intensité moteur relative et représente l'énergie mécanique apportée par l'extrudeuse à la matière extrudée.

Les granulés obtenus ont ensuite été séchés en turbétuve afin d'éliminer l'eau résiduelle.

Les 2 formulations ont ensuite subi une étape d'injection dans le but d'obtenir des éprouvettes normalisées. Les conditions d'injection ont été identiques pour les 2 matériaux.

Le tableau 3 présente les réglages de la presse à injecter qui ont été utilisés dans cette étude.

Tableau 3 : Paramètres d'injection sur presse ARBURG 100/420C/250

| | |
|---|---|
| Températures °C (Alimentation → Buse) | 40/55/60/80/110 |
| Empreinte | Eprouvette |
| Vitesse dosage (v401) m/min | 8,0 |
| Volume de dosage (V403) cm$^3$ | 14,3 |
| Contre Pression (p401) bar | 150 |
| Temps dosage (t402M) s | 3,9 |
| Pression injection (p301) bar | 800 |
| Debit injection (Q301) cm$^3$/s | 5 |
| Commutation (V311) cm$^3$ | 5 |
| Pression maintien (p321) bar | 650 |
| Débit maintien (Q321) cm3/s | 5 |
| Temps de maintien (t321) s | 30 |
| Temps refroidissement (t400) s | 25 |
| Temps cycle (t902M) s | 65,8 |
| Matelas (V321I) s | 1,3 |
| Vitesse éjecteur (v602) mm/s | 10 |
| Force éjecteur (F601) kN | 5 |
| Position buse | Décollée |
| Temperature moule °C | 18 |

Les efficacités de dégradation ont été évaluées en eau de source *Cristalline* Brièvement, pour chaque formulation, 4 éprouvettes normalisées (dont les masses ont été préalablement mesurées) ont été immergées dans un bac d'eau (eau de source référence *Cristalline*). Les bacs ont ensuite été placés dans une enceinte thermo régulée à 32°C et ventilée.

Une éprouvette a ensuite été prélevée après 1, 2, 3 et 6 mois et pesée (après étuvage à 103°C pendant 48h). La dégradabilité du matériau a été mesurée par perte de masse, calculée comme précisé précédemment).

Les résultats obtenus montrent une biodégradabilité totale du CAPA lorsque l'enzyme a été incorporée dans le matériau, dès 102 jours d'incubation des éprouvettes en eau, ceci démontrant que l'enzyme a résisté aux traitements thermiques infligés lors des étapes d'extrusion et d'injection.

Les inventeurs ont ainsi montré qu'une enzyme peut effectivement être incorporée dans un polymère dès la phase d'extrusion tout en conservant ses propriétés de dégradation du polymère.

Cette approche constitue donc une méthode simple, facile à mettre en oeuvre pouvant facilement être adaptée à bien d'autres couples polymère/enzymes permettant de contrôler la dégradation des matériaux en milieu naturel.

**Exemple 2 : Caractérisation des alliages de l'invention**

Les inventeurs ont comparé les propriétés mécaniques du CAPA seul, du CAPA mélangé à une charge végétale et d'un alliage constitué de CAPA et de la Lipase PS de *Pseudomonas cepacia* et obtenu selon le procédé de l'invention.

**Matériels et Méthodes**

1. Obtention des alliages

L'alliage CAPA/ Lipase PS de *Pseudomonas cepacia* a été obtenue à l'aide des matières premières suivantes:

Tableau 4 : Matières premières utilisées pour la formulation des alliages

| Nom | Fournisseur | Définition |
|---|---|---|
| CAPA 6506 (CAPA) | Solvay | Polycaprolactone en poudre |
| Blé | Amo | Farine native de blé référence « La Dorée » préalablement séchée |
| Lipase | Amano | Référence «PS SD » |

➢ *Etape de granulation par extrusion*

L'étape d'extrusion a été réalisée en 5 étapes, à l'aide d'une extrudeuse bi-vis corotative Clextral BC21

- introduction des différents éléments dans les proportions prévues grâce à un doseur pondéral et un doseur volumétrique ;
- fonte, malaxage, dégazage, mélangeage et mise sous pression de la matière dans les parties successives de l'extrudeuse ;
- sortie d'un jonc à travers une filière circulaire de 3 mm de diamètre ;
- refroidissement du jonc dans un bac d'eau froide de trois mètres de long puis « séchage » par de l'air froid puisé ;
- découpe sous forme de granulés réguliers par un système avec couteau tournant.

Quatre formulations ont ainsi été préparées (les % correspondent à des pourcentages massiques) :

- 100% CAPA;
- 98% CAPA + 2% lipase;
- 80% CAPA + 20% Blé;
- 55% CAPA + 45% Blé.

L'addition d'une charge végétale (farine de blé) est une alternative classique pour augmenter le taux de dégradation d'un polymère. En effet, le taux de dégradation maximal correspond à la dégradation de la charge végétale, le polymère utilisé comme liant n'étant en fait pas dégradé (voir ci-après).

Les granulés obtenus par extrusion ont ensuite été mis à sécher en turbétuve à 40°C pendant 12 heures afin d'éliminer l'eau résiduelle présente, due au passage dans le bac à eau. Les granulés séchés ont ensuite pu être injectés et caractérisés.

➢ *Etape d'injection*

L'étape d'injection permet de transformer les granulés obtenus par extrusion en des éprouvettes de caractérisation. Le principe de l'injection consiste à chauffer la matière plastique dans un fourreau pour l'amener à l'état fondu (phase de plastification) et à l'injecter sous pression dans un moule par l'intermédiaire d'un piston. La matière est solidifiée et en partie refroidie dans le moule avant d'en être éjectée. La presse utilisée est de marque ARBURG et de modèle Allrounder 1000-420C-250.

Les éprouvettes injectées correspondent au type 1 de la norme ISO 3167.

Certaines éprouvettes « 98% CAPA + 2% lipase » obtenues par injection ont subi un post-traitement avant caractérisation, consistant en un stockage dans un sac zip scellé placé en carton fermé pendant 1 mois à température ambiante, l'objectif de cette étape étant d'évaluer l'impact du stockage sur la stabilité du matériau, tant du point de vue de ses propriétés mécaniques que de sa capacité à se dégrader.

2. Détermination des caractéristiques mécaniques

➢ *Essai en traction*

Les caractéristiques en traction sont déterminées selon les recommandations décrites dans la norme internationale ISO/R 527 (détermination des caractéristiques en traction). Ces essais doivent être réalisés dans des conditions de température, d'humidité et de vitesse de séparation des mâchoires bien définies.

L'essai de traction consiste à imposer un allongement à une éprouvette de section initiale So et de longueur utile Lo. L'éprouvette est encastrée à ses deux extrémités dans des mors. L'une de ces mâchoires, mobile, est reliée à un système d'entraînement à vitesse de déplacement linéaire. La mesure des efforts se fait à l'aide d'un capteur de force électronique (10 kN).

Les renseignements relevés à partir de ces essais sont au nombre de trois :

- la contrainte de traction maximale (Cmax TR ; MPa),
- l'allongement à la rupture (en %),
- le module de Young en traction (en Mpa).

Comme indiqué dans la norme, les essais sont réalisés sur cinq éprouvettes de chaque lot de matière et les résultats présentés sont la moyenne de ces cinq déterminations. La vitesse de déplacement est fixée à 50 mm/min et le module de traction est calculé entre 10 et 50% de la valeur de la contrainte maximale. La machine de traction utilisée est commercialisée par ZWICK.

➢ *Choc Charpy*

Cette méthode permet d'étudier le comportement d'éprouvettes définies, soumises à des contraintes de choc pour en estimer la fragilité ou la ténacité. Pour ces essais, les inventeurs ont utilisé les éprouvettes de type 1 précédemment décrites, sans entaille.

Le matériel utilisé est un mouton-pendule d'une énergie de 15 Joules de marque ZWICK. L'unité est pilotée par un logiciel qui enregistre les valeurs mesurées et les analyses. Lors de l'essai, l'éprouvette est placée horizontalement devant les appuis et percutée par le pendule en son milieu. La résistance au choc Charpy correspond à l'énergie absorbée par la rupture d'une éprouvette rapportée à sa section droite avant essai (Résilience).

3. Caractérisations rhéologiques : indice de fluidité à chaud (MFR)

La norme ISO 1133 prescrit une méthode de détermination de l'indice de fluidité à chaud (ou MFR) des thermoplastiques dans des conditions de température et de pression définies. Cet essai permet de déterminer le grade du polymère testé et nous informe sur l'aptitude du polymère à s'écouler à travers une filière pour une température donnée (ce qui est un paramètre important pour l'injection).

L'appareil utilisé est un plastomètre d'extrusion de marque ZWICK relié à une balance de précision et piloté par un logiciel spécifique pouvant déterminer le MFR exprimé en g/10 minutes.

Lors d'un essai, le mélange, chauffé dans le cylindre vertical, est extrudé à travers la filière au moyen d'une charge fixée sur un piston. Cinq extrudas sont coupés à des intervalles de temps réguliers, récupérés puis pesés. Les paramètres à fixer sont donc la température du cylindre, le poids de la charge et le temps entre deux coupes.

4. Test de dégradation en eau

Pour chaque formulation, 4 éprouvettes normalisées (dont les masses séchées à 103°C ont été préalablement mesurées) sont immergées dans un bac d'eau (eau de source référence *Cristalline,* Leclerc). Les bacs sont placés dans une enceinte thermo régulée à 32 °C et ventilée. Une éprouvette est ensuite prélevée après 1, 2, 3 et 4 mois et pesée (après étuvage à 103°C pendant 48h). La dégradabilité du matériau est mesurée par perte de masse calculée de la manière suivante :

$$Perte\ (\%) = (perte\ de\ masse\ totale - perte\ d'eau\ 103°C)\ x100$$

**Résultats**

1. Caractéristiques mécaniques et rhéologie

Les résultats obtenus sont présentés dans le ci-dessous:

Tableau 5 : Caractéristiques des matériaux

| Formulation (% massique) | Post -traitement | Résilience Kj/m$^2$ | MFR 150°C/2, 16kg | Essais en traction | | | | Densité (g/cm3) | % de dégradation à 3 mois |
| --- | --- | --- | --- | --- | --- | --- | --- | --- | --- |
| | | | | Cmax TR Mpa | Allongement à la rupture (%) | Module de Young (Mpa) | Rupture en fin de traction | | |
| 100% CAPA | | 23 | 14 | 23 | 533 | 400 | Pas de rupture | 1.11 | 1 |
| 98% CAPA + 2% lipase | | 24 | 14 | 23 | 530 | 450 | Pas de rupture | 1.14 | 71 |
| 98% CAPA + 2% lipase | Stockage 1 mois dans l'obscurité en sac scellé | ND | ND | 26 | 530 | 500 | Pas de rupture | 1.14 | 77 |
| 80% CAPA + 20% Blé | | 24 | 11 | 19 | 532 | 500 | Pas de rupture | 1.16 | 18 |
| 55% CAPA + 45% Blé | | 21 | 4 | 9 | 286 | 675 | Rupture | 1.22 | 42 |

Ce tableau indique que l'addition d'enzyme ne modifie pas les propriétés mécaniques du CAPA testées ici, à savoir la résilience, la MFR ou l'ensemble des paramètres en traction. Ceci est en contraste très net avec les polymères constitués de 55% CAPA + 45% Blé, qui voient l'ensemble des propriétés mécaniques dégradées par rapport au CAPA seul. Le mélange 80% CAPA + 20% Blé permet, quant à lui, de conserver des propriétés similaire à celle du CAPA seul.

Aussi, l'alliage de l'invention est hautement adapté pour améliorer la dégradation du CAPA, tout en conservant ses propriétés.

2. Dégradation en milieux aqueux

Les résultats indiquent que le CAPA seul ne se dégrade pas lorsqu'il est immergé dans un environnement aqueux. Si l'addition d'une charge végétale de 20% permet d'améliorer la dégradation de l'alliage, il n'en reste pas moins que le taux maximal atteint moins de 20%. Comme cela a déjà été indiqué, cette dégradation correspond à la dégradation de la farine de blé, alors que le matériau demeure, quant à lui, inaltéré.

Une amélioration de la biodégradation est donc directement corrélée à une augmentation du pourcentage de la charge végétale dans l'alliage avec, pour corollaire, une altération des propriétés mécaniques.

Les résultats indiquent également que l'addition d'enzymes est hautement plus avantageuse que l'addition d'une charge végétale, puisqu'elle induit la dégradation du CAPA (environ 70 % après 3 mois en eau), tout en conservant les propriétés mécaniques du matériau pur.

Les inventeurs ont ainsi réussi à montrer que les compositions obtenues présentent une meilleure dégradabilité qu'avec une charge végétale, et ceci en maintenant les propriétés mécaniques du polymère.

Enfin, le stockage dans l'obscurité et sac scellé du polymère 98% CAPA + 2% lipase n'altère ni les propriétés mécaniques, ni sa capacité à se dégrader (comparaison des comportements des polymères 98% CAPA + 2% lipase après post-traitement ou non). L'alliage de l'invention est donc stable et ne se dégrade pas lorsqu'il n'est pas en solution et est ainsi approprié pour le stockage.

En conclusion, l'ensemble des résultats indiquent que le procédé de préparation, d'alliages polymère/enzymes selon l'invention est une solution industrielle particulièrement attractive pour le développement de matériau à courte durée de vie, tout en diminuant les impacts négatifs sur l'environnement, inhérents à la dissémination de résidus dans le milieu naturel.

**Exemple 3. Impact de la température sur la biodégradation de la polycaprolactone (CAPA)**

Afin de démontrer que la température de transformation de la polycaprolactone n'a pas d'impact sur sa biodégradation, la polycaprolactone a été extrudée à 170°C et sa biodégradation a été évaluée.

3.1. Etape de granulation par extrusion de la polycaprolactone à 170°C

L'étape d'extrusion est réalisée comme défini dans l'exemple 1 (voir section Matériels et Méthodes 1), si ce n'est que la température d'extrusion dans cet exemple est de 170°C et la formulation est composée de 100% CAPA (sans enzyme). Les paramètres d'extrusion sont présentés dans le tableau 6.

**Tableau 6. Paramètres d'extrusion réalisée dans cette étude.**

| Formulation | Températures d'extrusion (Zone 1 à filière) | Vitesse BC 21, T/min | Pression, bars | Couple (%) |
|---|---|---|---|---|
| 100%m CAPA 6506 | 20-80-7*170 | 200 | 42 | 38 |

3.2. Injection

Les granulés de 100% CAPA (cf. 100%m CAPA 6506) ont ensuite subi une étape d'injection dans le but d'obtenir des éprouvettes normalisées. L'étape d'injection est réalisée comme décrit dans l'exemple 1.

Le tableau 7 présente les réglages de la presse à injecter qui ont été utilisés dans cette étude.

**Tableau 7. Paramètres d'injection sur presse ARBURG 100/420C/250.**

| Températures °C (Alimentation → Buse) | 40/55/60/80/100 |
|---|---|
| Empreinte | Eprouvette |
| Vitesse dosage (v401) m/min | 8,0 |

(suite)

| | |
|---|---|
| Températures °C (Alimentation → Buse) | 40/55/60/80/100 |
| Empreinte | Eprouvette |
| Volume de dosage (V403) cm$^3$ | 19 |
| Contre Pression (p401) bar | 150 |
| Temps dosage (t402M) s | 4,5-7,5 |
| Pression injection (p301) bar | 800 |
| Debit injection (Q301) cm3/s | 5 |
| Commutation (V311) cm$^3$ | 5 |
| Pression maintien (p321) bar | 650 |
| Débit maintien (Q321) cm3/s | 5 |
| Temps de maintien (t321) s | 30 |
| Temps refroidissement (t400) s | 20 |
| Temps cycle (t902M) s | 60 |
| Matelas (V321I) s | 1± 0,5 |
| Vitesse éjecteur (v602) mm/s | 400 |
| Force éjecteur (F601) kN | 20 |
| Position buse | Décollée |
| Temperature moule °C | Froid direct, 5°C |

3.3. Mesure de la biodégradation

➢ Dégradation dans l'eau 30°C : suivi de la perte de masse

La dégradation a été évaluée en eau de source *Cristalline.* 4 éprouvettes normalisées (dont les masses ont été préalablement mesurées) ont été immergées dans un bac d'eau. Les bacs ont ensuite été placés dans une enceinte thermo régulée à 32 °C et ventilée.

Une éprouvette a ensuite été prélevée après 15 jours, 1, 2 et 3 mois et pesée (après étuvage à 103°C pendant 48h). La dégradabilité du matériau a été mesurée par perte de masse, calculée comme précisé dans l'exemple 1.

Toute comme pour la formulation 100%m CAPA 6506 (voir Exemple 2.), la «perte de masse» des éprouvettes réalisées à partir de la formulation 100%m CAPA 6506-170 ne montre aucune dégradation après 2 mois. Le suivi de la dégradation dans l'eau à 32°C permet de démontrer que le CAPA seul ne se dégrade pas au cours du temps dans l'eau, lorsqu'il est extrudé à des températures de 80°C jusqu'à 170°C.

**Exemple 4. Inclusion de 2% Lipase PS de *Pseudomonas cepacia* dans 98% CAPA à 100°C**

4.1. Inclusion de l'enzyme

Les inventeurs ont réalisé une extrusion de la polycaprolactone en incorporant ladite enzyme. Pour cela un mélange de 98% massique de polymère et de 2% massique d'enzyme a été placé dans un doseur. Les quantités utilisées dans le mélange sont présentées ici (voir tableau 8).

**Tableau 8. Formulation réalisée dans cette étude**

| Formulation (% massique) | Masse d'enzyme (g) dans doseur | Masse CAPA 6506 P (g) dans doseur |
|---|---|---|
| 98%m CAPA 6506 + 2%m lipase | 30 | 1500 |

4.1.a. Extrusion

L'extrusion a été réalisée à 100°C. L'étape d'extrusion était accomplie comme décrit dans l'exemple 1 (voir section Matériels et Méthodes 1).

Les paramètres de l'extrusion sont présentés dans le tableau 9.

**Tableau 9. Paramètres d'extrusion réalisée dans cette étude.**

| Formulation | Températures d'extrusion (Zone 1 à filière) | Vitesse BC 21, T/min | Pression, bars | Couple (%) |
|---|---|---|---|---|
| 98%m CAPA 6506 + 2%m lipase | *20-80-7\*100* | 200 | 65 | 53 |

4.2. Injection

La formulation a ensuite subi une étape d'injection dans le but d'obtenir des éprouvettes normalisées. Les réglages de la presse à injecter qui ont été utilisés dans cette étude sont les mêmes utilisées dans l'exemple 3 (voir tableau 7).

4.3. Mesure de biodégradation

Les efficacités de dégradation ont été évaluées par la dégradation dans l'eau.

➢ Dégradation dans l'eau 30°C : suivi de la perte de masse

La dégradation a été évaluée en eau de source *Cristalline* comme dans l'exemple 3 (voir section 3.3).
Une éprouvette a ensuite été prélevée après 15 jours, 1, 2 et 3 mois et pesée (après étuvage à 103°C pendant 48h). La dégradabilité du matériau a été mesurée par perte de masse, calculée comme précisé dans l'exemple 1.
Les résultats obtenus montrent une biodégradabilité avec une perte de masse de 54% après 2 mois. Ceci démontre que l'enzyme a résisté aux traitements thermiques infligés lors des étapes d'extrusion et d'injection.
Les inventeurs ont ainsi montré qu'une enzyme peut effectivement être incorporée dans un polymère dès la phase d'extrusion à 100 °C tout en conservant ses propriétés de dégradation du polymère.

**Exemple 5. Inclusion de 2% Lipase PS dans 98% CAPA à 120°C**

5.1. Inclusion de l'enzyme

Les inventeurs ont réalisé une extrusion de la polycaprolactone en incorporant ladite enzyme. Pour cela le polymère a été placé dans un doseur et l'enzyme dans un autre, tous les deux sous forme de poudre.
Dans les expériences qui sont présentées ici, les débits de ces deux doseurs ont été réglés de manière à obtenir un pourcentage d'enzyme dans le matériau de 2 % (m/m) (voir tableau 10).

**Tableau 10. Formulation réalisée dans cette étude**

| Formulation (% massique) | Masse d'enzyme (g) dans doseur | Masse CAPA 6506 P (g) dans doseur |
|---|---|---|
| 98%m CAPA 6506 + 2%m lipase | 30 | 1500 |

5.1.a. Extrusion

L'alliage entre le polymère et l'enzyme a été réalisée par extrusion à 120°C L'étape d'extrusion était accomplie comme décrit dans l'exemple 1 (voir section Matériels et Méthodes 1).
Les paramètres d'extrusion sont présentés dans le tableau 11.

**Tableau 11. Paramètres d'extrusion réalisée dans cette étude.**

| Formulation | Températures d'extrusion (Zone 1 à filière) | Vitesse BC 21, T/min | Pression, bars | Couple (%) |
|---|---|---|---|---|
| 98%m CAPA 6506 + 2%m lipase | *20-80-7\*120* | 200 | 65 | 55 |

### 5.2. Injection

La formulation a ensuite subi une étape d'injection dans le but d'obtenir des éprouvettes normalisées. Les conditions d'injection ont été identiques que pour l'exemple 3 (voir tableau 7).

### 5.3. Mesure de Biodégradation

Les efficacités de dégradation ont été évaluées par la dégradation dans l'eau.

➢ Dégradation dans l'eau 30°C : suivi de la perte de masse

La dégradation a été évaluée en eau dans les mêmes conditions décrites dans l'exemple 3.
Les résultats obtenus montrent une biodégradabilité avec une perte de masse de 27,5% après 2 mois. Ceci démontre que l'enzyme a résisté aux traitements thermiques infligés lors des étapes d'extrusion et d'injection.
Les inventeurs ont ainsi montré qu'une enzyme peut effectivement être incorporée dans un polymère dès la phase d'extrusion à 120 °C tout en conservant ses propriétés de dégradation du polymère.

### Exemple 6. Inclusion de 2% Lipase PS dans 98% CAPA à 140°C

Les inventeurs ont réalisé une extrusion de la polycaprolactone en incorporant ladite enzyme. Pour cela le polymère a été placé dans un doseur et l'enzyme dans un autre, tous les deux sous forme de poudre.
Dans les expériences qui sont présentées ici, les débits de ces deux doseurs ont été réglés de manière à obtenir un pourcentage d'enzyme dans le matériau de 2% (m/m) (voir tableau 12).

**Tableau 12. Formulation réalisée dans cette étude**

| Formulation (% massique) | Masse d'enzyme (g) dans doseur | Masse CAPA 6506 P (g) dans doseur |
|---|---|---|
| 98%m CAPA 6506 + 2%m lipase | 30 | 1500 |

### 6.1.a. Extrusion

L'extrusion a été réalisée à 140°C. L'étape d'extrusion était accomplie comme il est décrit dans l'exemple 1 (voir section Matériels et Méthodes 1).
Les paramètres d'extrusion sont présentés dans le tableau 13.

**Tableau 13. Paramètres d'extrusion réalisée dans cette étude.**

| Formulation | Températures d'extrusion (Zone 1 à filière) | Vitesse BC 21, T/min | Pression, bars | Couple (%) |
|---|---|---|---|---|
| 98%m CAPA 6506 + 2%m lipase-140 | *20-80-7\*140* | 200 | 59 | 48 |

### 6.2. Injection

La formulation a ensuite subi une étape d'injection dans le but d'obtenir des éprouvettes normalisées. Les réglages de la presse à injecter qui ont été utilisés dans cette étude sont les mêmes utilisées dans l'exemple 3 section 3.2 (voir tableau 7).

6.3. Mesure de Biodégradation

Les efficacités de dégradation ont été évaluées par la dégradation dans l'eau.

> Dégradation dans l'eau 30°C : suivi de la perte de masse

La dégradation a été évaluée en eau de source *Cristalline* comme dans l'exemple 3 (voir section 3.3).
Une éprouvette a ensuite été prélevée après 15 jours, 1, 2 et 3 mois et pesée (après étuvage à 103°C pendant 48h). La dégradabilité du matériau a été mesurée par perte de masse, calculée comme précisé dans l'exemple 1 (voir section Résultat 1.a.).
Les résultats obtenus montrent une biodégradabilité avec une perte de masse de 4,5% après 2 mois. Ceci démontre que l'enzyme a résisté aux traitements thermiques infligés lors des étapes d'extrusion et d'injection.
Les inventeurs ont ainsi montré qu'une enzyme peut effectivement être incorporée dans un polymère dès la phase d'extrusion à 140 °C tout en conservant ses propriétés de dégradation du polymère.
En conclusion, les inventeurs ont exemplifié le fait que le procédé de l'invention permet d'obtenir un alliage qui se dégrade à différentes températures d'extrusion, en particulier à 80°C, 100°C, 120°C et 140°C.

**Exemple 7 : Méthodes d'introduction de la lipase**

Afin de démontrer l'importance de l'inclusion de la lipase par extrusion sur la biodégradation du matériau correspondant, différentes méthodes d'introduction de la lipase ont été réalisées sur une polycaprolactone.

1 : Introduction de la lipase par extrusion (Essai 7a)

Un mélange de 98%m de polycaprolactone (CAPA 6506) et de 2%m de lipase PS a été extrudé à 65°C dans les conditions décrites dans l'exemple 1 (voir section Matériels et Méthodes 1).
Les paramètres d'extrusion sont présentés dans le tableau 14.

**Tableau 14. Paramètres d'extrusion réalisée dans cette étude.**

| Formulation | Températures d'extrusion (Zone 1 à filière) | Vitesse BC 21, T/min | Pression, bars | Couple (%) |
|---|---|---|---|---|
| 98%m CAPA 6506 + 2%m lipase | *20-20-40-6\*65* | 250 | 93 | 63 |

La formulation a ensuite subi une étape d'injection dans le but d'obtenir des éprouvettes normalisées. Les réglages de la presse à injecter sont les mêmes que ceux utilisés dans l'exemple 3 (voir tableau 7).
L'efficacité de dégradation des pièces injectées référence 7a a ensuite été évaluée par la dégradation dans l'eau, selon la méthode décrite dans l'exemple 3.

2. Introduction de la lipase par pulvérisation à la surface de pièces injectées de polycaprolactone (Essai 7b)

Une polycaprolactone CAPA6506 non extrudée a subi une étape d'injection dans le but d'obtenir des éprouvettes normalisées. Les réglages de la presse à injecter sont les mêmes que ceux utilisés dans l'exemple 3 (voir tableau 7).
Une solution aqueuse de lipase PS a ensuite été pulvérisée à la surface des pièces injectées de polycaprolactone jusqu'à obtenir l'équivalent d'un mélange de 98%m de polycaprolactone et de 2%m de lipase.
L'efficacité de dégradation des pièces injectées a ensuite été évaluée par la dégradation dans l'eau, selon la méthode décrite dans l'exemple 3.

3. Introduction de la lipase dans l'eau utilisée au test de dégradation dans l'eau (Essai 7c)

Une polycaprolactone CAPA6506 non extrudée a subi une étape d'injection dans le but d'obtenir des éprouvettes normalisées. Les réglages de la presse à injecter sont les mêmes que ceux utilisés dans l'exemple 3 (voir tableau 7).
L'efficacité de dégradation des pièces injectées référence 7a a ensuite été évaluée par la dégradation dans l'eau ensemencée de lipase PS, selon la méthode décrite dans l'exemple 3. La quantité de lipase ajoutée équivaut à un mélange de 98%m de polycaprolactone et de 2%m de lipase PS.
Les pertes en masse des formulations 7a, 7b et 7c sont reprises dans le tableau ci-dessous :

**Tableau 15. Perte de masse dans l'eau des échantillons 7a, 7b et 7c.**

| Référence | Méthode d'introduction de la lipase PS | Perte de masse (%massique) dans l'eau après | | | |
|---|---|---|---|---|---|
| | | 15 jours | 1 mois | 2 mois | 3 mois |
| 7a | Extrusion | 16 | 34 | 64 | 86 |
| 7b | Aspersion de surface | 17 | 21 | 21 | 21 |
| 7c | Ensemencement de l'eau utilisée en dégradation | 15 | 21 | 31 | 32 |

Ces résultats mettent en lumière le fait que lorsque la lipase est introduite par aspersion ou dans la solution de dégradation, la dégradation est moins importante que lorsque la lipase est incluse dans l'alliage.

En d'autres termes, les inventeurs ont montré que l'étape d'extrusion permet d'obtenir un alliage présentant une meilleure dégradabilité que l'étape de dispersion/solution de la lipase.

**Exemple 8 : Essais complémentaires**

Les matières premières utilisées pour ces essais complémentaires sont comme suit :

- Polycaprolactone CAPA 6506

- Farine de bois Arbocel C320 fourni par Rettenmaier ; et

- Farine de blé La Dorée fourni par AMO

Différents alliages de polycaprolactone, de lipase PS et/ou de charge végétale (farine de blé ou farine de bois) ont été préparés par extrusion dans les conditions décrites dans l'exemple 1 (voir section Matériels et Méthodes) :

**Tableau 16. Composition des différents alliages obtenus par extrusion dans cette étude.**

| Référence | Composition |
|---|---|
| 7a | 98%m CAPA + 2%m lipase |
| 8 | 55%m CAPA + 45%m farine de blé |
| 9 | 80%m CAPA + 20%m farine de blé |
| 10 | 100% CAPA |
| 11 | 55%m CAPA + 45%m farine de bois |

Les paramètres d'extrusion sont présentés dans le tableau 17.

**Tableau 17. Paramètres d'extrusion réalisée dans cette étude.**

| Températures d'extrusion (Zone 1 à filière) | Vitesse BC 21, T/min |
|---|---|
| *20-20-40-6*65* | 250 |

Les différents alliages obtenus ont ensuite subi une étape d'injection dans le but d'obtenir des éprouvettes normalisées. Les réglages de la presse à injecter sont les mêmes que ceux utilisés dans l'exemple 3 (voir tableau 7).

Les efficacités de dégradation des pièces injectées référence 7a, 8, 9, 10 et 11 ont ensuite été évaluées par la dégradation dans l'eau, selon la méthode décrite dans l'exemple 3. Ces efficacités sont présentées dans le tableau ci-après :

**Tableau 18.Perte de masse des échantillons 7a, 8, 9, 10 et 11.**

| Référence | Composition | Perte de masse (%massique) dans l'eau après | | | |
|---|---|---|---|---|---|
| | | 15 jours | 1 mois | 2 mois | 3 mois |
| 7a | 98%m CAPA + 2%m lipase | 16 | 34 | 64 | 86 |

(suite)

| Référence | Composition | Perte de masse (%massique) dans l'eau après | | | |
|---|---|---|---|---|---|
| | | 15 jours | 1 mois | 2 mois | 3 mois |
| 8 | 55%m CAPA + 45%m farine de blé | <1 | 3 | 6 | 9 |
| 9 | 80%m CAPA + 20%m farine de blé | <1 | 4 | 6 | 7 |
| 10 | 100% CAPA | <1 | <1 | <1 | <1 |
| 11 | 55%m CAPA + 45%m farine de bois | <1 | <1 | <1 | <1 |

Les inventeurs ont ainsi montré qu'afin d'obtenir une forte biodégradabilité, l'addition d'enzymes est hautement plus avantageuse que l'addition d'une charge végétale.

**Revendications**

1. Procédé de préparation d'un alliage polymère solide/entités biologiques comprenant les étapes selon lesquelles:

   - on sélectionne un polymère parmi le polycaprolactone, l'acide polylactique, le polyéthylène téréphtalate, le poly(triméthylène téréphthalate), un polyhydroxyalkanoate en C1-C6, l'acétate de cellulose, le poly(butylène adipate-co-téréphtalate), le poly(butylène succinate), le polyamide PA 11, et leurs mélanges;
   - on sélectionne des entités biologiques parmi les enzymes et les microorganismes aptes à dégrader ledit polymère en condition aqueuse;
   - on réalise l'alliage en mélangeant le polymère et les entités biologiques, lors d'un traitement thermique conduit à une température T supérieure à la température ambiante, à laquelle le polymère est partiellement ou totalement fondu, lesdites entités biologiques permettant de dégrader ledit polymère dans l'alliage.

2. Procédé selon la revendication 1, **caractérisé en ce que** ladite température T est comprise entre la température de transition vitreuse et la température de fusion dudit polymère.

3. Procédé selon la revendication 1, **caractérisé en ce que** ladite température T est la température de fusion dudit polymère.

4. Procédé selon l'une quelconque des revendications 1 à 3, **caractérisé en ce que** ledit traitement thermique consiste en une opération choisie parmi l'extrusion, l'injection, le thermoformage, le rotomoulage, la compression, le calandrage, la chaudronnerie, l'enduction, la stratification, l'expansion, la pultrusion, l'extrusion-soufflage, l'extrusion-gonflage, et la compression-granulation, de préférence l'extrusion.

5. Procédé selon l'une quelconque des revendications 1 à 4, **caractérisé en ce que** le ratio entités biologiques/polymère en poids est compris entre environ 0,1% et environ 10%, préférentiellement entre environ 0,5% et environ 8%, préférentiellement entre 30 environ 1% et environ 6%, préférentiellement entre environ 1% et environ 5%, préférentiellement entre environ 1% et environ 4%, préférentiellement entre environ 1% et environ 3%, préférentiellement entre environ 1,5% et environ 3%, et encore plus préférentiellement ce ratio est d'environ 2%.

6. Procédé selon l'une quelconque des revendications 1 à 5, **caractérisé en ce que** lesdites entités biologiques sont des enzymes thermorésistantes choisies de préférence parmi: la lipase PS de *Pseudomonas cepacia,* la lipase B de *Candida antartica,* la protéinase K, une polyhydroxyalkanoate en C1-C6 dépolymérase et leurs mélanges.

7. Procédé l'une quelconque des revendications 1 à 5, **caractérisé en ce que** lesdites entités biologiques sont des enzymes thermostabilisées, choisies de préférence parmi les enzymes encapsulées dans des nanocapsules constituées de la même matière que ledit polymère, les enzymes encapsulées dans des molécules cages et les enzymes agrégées entre elles.

8. Procédé selon l'une quelconque des revendications 1 à 7, **caractérisé en ce que** ledit polymère est le polycaprolactone et l'entité biologique comprend une lipase, préférentiellement une amanolipase PS de *Pseudomonas cepacia.*

9. Procédé de préparation d'un alliage polymère solide/entités biologiques comprenant une étape d'extrusion d'un polymère avec des entités biologiques choisies parmi les enzymes et les microorganismes dégradant ledit polymère, dans un ratio en poids entités biologiques/polymère compris entre 1% et 5%, à une température à laquelle le polymère est partiellement ou totalement fondu, ledit polymère étant choisi parmi le polycaprolactone, l'acide polylactique, le polyéthylène téréphtalate, le poly(triméthylène téréphthalate), un polyhydroxyalkanoate en C1-C6, l'acétate de cellulose, le poly(butylène adipate-co-téréphtalate), le poly(butylène succinate), le polyamide PA 11, et leurs mélanges lesdites entités biologiques permettant de dégrader ledit polymère dans l'alliage.

**Patentansprüche**

1. Verfahren zur Herstellung einer Legierung aus festem Polymer/biologischen Einheiten, umfassend die folgenden Schritte:

- Auswählen eines Polymers aus Polycaprolacton, Polymilchsäure, Polyethylenterephthalat, Poly(trimethylenterephthalat), einem C1-C6-Polyhydroxyalkanoat, Celluloseacetat, Poly(butylenadipat-co-terephthalat), Poly(butylensuccinat), Polyamid PA 11 und ihren Gemischen;
- Auswählen der biologischen Einheiten aus Enzym und Mikroorganismen, die für einen Abbau besagten Polymers unter wässrigen Bedingungen geeignet sind;
- Herstellen der Legierung durch Mischen des Polymers und der biologischen Einheiten, während eine thermische Behandlung zu einer höheren Temperatur T als die Raumtemperatur führt, bei der das Polymer partiell oder vollständig geschmolzen ist, wobei besagte biologische Einheiten einen Abbau besagten Polymers in der Legierung erlauben.

2. Verfahren gemäß Anspruch 1, **dadurch gekennzeichnet, dass** besagte Temperatur T zwischen der Glasübergangstemperatur und der Schmelztemperatur besagten Polymers liegt.

3. Verfahren gemäß Anspruch 1, **dadurch gekennzeichnet, dass** besagte Temperatur T die Schmelztemperatur besagten Polymers ist.

4. Verfahren gemäß einem der Ansprüche 1 bis 3, **dadurch gekennzeichnet, dass** besagte thermische Behandlung aus einem Vorgang besteht, ausgewählt aus Extrusion, Injektion, Warmformen, Rotationsformen, Komprimieren, Kalandrieren, Tiefziehen, Beschichten, Laminieren, Expansion, Pultrusion, Extrusionsblasen, Blasextrusion und Kompressionsgranulation, bevorzugt ausgewählt aus Extrusion.

5. Verfahren gemäß einem der Ansprüche 1 bis 4, **dadurch gekennzeichnet, dass** das Gewichtsverhältnis von biologischen Einheiten/Polymer zwischen ungefähr 0,1% und ungefähr 10%, bevorzugt zwischen ungefähr 0,5% und ungefähr 8%, bevorzugt zwischen ungefähr 1% und ungefähr 6%, bevorzugt zwischen ungefähr 1% und ungefähr 5%, bevorzugt zwischen ungefähr 1% und ungefähr 4%, bevorzugt zwischen ungefähr 1% und ungefähr 3%, bevorzugt zwischen ungefähr 1,5% und ungefähr 3% liegt und bevorzugter dieses Verhältnis ungefähr 2% beträgt.

6. Verfahren gemäß einem der Ansprüche 1 bis 5, **dadurch gekennzeichnet, dass** besagte biologische Einheiten thermoresistente Enzyme sind, bevorzugt ausgewählt aus Lipase PS von *Pseudomonas cepacia,* Lipase B von *Candida antarctica,* Proteinase K, einer C1-C6-Polyhydroxyalkanoat-Depolymerase und ihren Gemischen.

7. Verfahren gemäß einem der Ansprüche 1 bis 5, **dadurch gekennzeichnet, dass** besagte biologische Einheiten thermostabile Enzyme sind, bevorzugt ausgewählt aus Enzymen, die in Nanokapseln verkapselt sind, die aus demselben Material wie besagtes Polymer bestehen, Enzymen, die in Käfigmolekülen verkapselt sind, und miteinander aggregierten Enzymen.

8. Verfahren gemäß einem der Ansprüche 1 bis 7, **dadurch gekennzeichnet, dass** besagtes Polymer Polycaprolacton ist und die biologische Entität eine Lipase, bevorzugt eine Amanolipase PS von *Pseudomonas cepacia,* umfasst.

9. Verfahren zur Herstellung einer Legierung aus festem Polymer/biologischen Einheiten, umfassend einen Schritt der Extrusion eines Polymers mit biologischen Einheiten, ausgewählt aus Enzymen und Mikroorganismen, die besagtes Polymer abbauen, in einem Gewichtsverhältnis von biologischen Einheiten/Polymer zwischen 1% und 5%, bei einer Temperatur, bei der das Polymer partiell oder vollständig geschmolzen ist, wobei besagtes Polymer aus Polycaprolacton, Polymilchsäure, Polyethylenterephthalat, Poly(trimethylenterephthalat), einem C1-C6-Polyhydroxyalkanoat, Celluloseacetat, Poly(butylenadipat-co-terephthalat), Poly(butylensuccinat), Polyamid PA 11 und ihren Gemischen ausgewählt ist, wobei besagte biologische Einheiten einen Abbau besagten Polymers in der Legierung erlauben.

**Claims**

1. A process for preparing a solid polymer/biological entities alloy, comprising the following steps

   - selecting a polymer selected from polycaprolactone, polylactic acid, polyethylene terephthalate, poly(trimethylene terephthalate), a $C_1$-$C_6$ polyhydroxyalkanoate, cellulose acetate, poly(butylene adipate-co-terephthalate), poly(butylene succinate) and polyamide PA11, and mixtures thereof;
   - selecting biological entities selected chosen from enzymes and microorganisms that degrade said polymer in aqueous conditions;
   - preparing the alloy by mixing the polymer and biological entities, during a heat treatment performed at a temperature T above room temperature, at which the polymer is in a partially or totally molten state, said biological entities being able to degrade said polymer in said alloy.

2. The process as claimed in claim 1, wherein said temperature T is between the glass transition temperature and the melting point of said polymer.

3. The process as claimed in claim 1, wherein said temperature T is the melting point of said polymer.

4. The process as claimed in any one of claims 1 to 3, wherein said heat treatment consists of an operation chosen from extrusion, injection-molding, thermoforming, rotary molding, compression, calendering, ironing, coating, stratification, expansion, pultrusion, extrusion blow-molding, extrusion-swelling and compression-granulation, preferably extrusion.

5. The process as claimed in any one of claims 1 to 4, wherein the biological entities/polymer weight ratio is between about 0.1% and about 10%., preferably between about 0.5% and about 8%, preferably between about 1% and about 6%, preferably between about 1% and about 5%, preferably about 1% and about 4%, preferably about 1% and about 3%, preferably about 1.5% and about 3%, and even more preferably said ratio is about 2%.

6. The process as claimed in any one of claims 1 to 5, wherein said biological entities are heat-resistant enzymes preferably chosen from: lipase PS from *Pseudomonas cepacia,* lipase B from *Candida antartica,* proteinase K and a $C_1$-$C_6$ polyhydroxyalkanoate depolymerase, and mixtures thereof.

7. The process as claimed in any one of claims 1 to 5, wherein said biological entities are heat-stabilized enzymes preferably chosen from enzymes encapsulated in nanocapsules consisting of the same material as said polymer, enzymes encapsulated in cage molecules and enzymes aggregated together.

8. The process as claimed in any one of claims 1 to 7, wherein said polymer is polycaprolactone and said biological entity comprises a lipase, preferably an amanolipases PS from *Pseudomonas cepacia.*

9. A process for preparing a solid polymer/biological entities alloy, comprising a step of extruding a polymer with biological entities selected from the group consisting of enzymes and microorganisms that degrade said polymer, wherein the biological entities/polymer weight ratio is between about 1% and 5%, at a temperature at which the polymer is in a partially or totally molten state, said polymer being selected from polycaprolactone, polylactic acid, polyethylene terephthalate, poly(trimethylene terephthalate), a $C_1$-$C_6$ polyhydroxyalkanoate, cellulose acetate, poly(butylene adipate-co-terephthalate), poly(butylene succinate) and polyamide PA11, and mixtures thereof, said biological entities being able to degrade said polymer in said alloy.